# NEUE EUROPÄISCHE PATENTSCHRIFT

(11) **EP 0 657 529 B2**
(45) Veröffentlichungstag und Bekanntmachung des Hinweises auf die Entscheidung über den Einspruch: **16.11.2005**
(45) Hinweis auf die Patenterteilung: 23.05.2001
(21) Anmeldenummer: 94119369.0
(22) Anmeldetag: 08.12.1994
(51) Int. Cl.: C12M 1/12, C12P 7/26, C12P 1/00

(54) **Entfärbung von Fermentationslösungen**
Decolorizing fermentation solutions
Décoloration de solutions de fermentation

(30) Priorität: 11.12.1993 DE 4342345
(43) Veröffentlichungstag der Anmeldung: 14.06.1995
(73) Patentinhaber: Merck Patent GmbH, 64293 Darmstadt (DE)
(72) Erfinder: Ohrem, Hans Leonhard, D-64331 Weiterstadt (DE); Büttgenbach, Lutz, Dr., D-64372 Ober-Ramstadt (DE)

(56) Entgegenhaltungen:
- WO-A-91/04342
- DD-A- 139 515
- US-A- 4 758 347
- CHEMICAL ABSTRACTS, vol. 112, no. 13, 26.März 1990 Columbus, Ohio, US; abstract no. 117391, TOSHIHIRO M. & TAKASHI K.: "Separation and recovery of fermented meso-erythritol with ultrafiltration membranes." XP002058980 & JP 01 215 293 A (MITSUBISHI KASEI CORP.: NIKKEN CHEMICALS C. LTD.) 29.August 1989
- BRENES BALBUENA M. ET AL.: "Regeneration of spanish style green table olive brines by ultrafiltration." JOURNAL OF FOOD SCIENCE, Bd. 53, Nr. 6, 1988, Seiten 1733-1736, XP002058979

## Beschreibung

Die Erfindung betrifft ein Verfahren zum einfachen Abtrennen der gefärbten Bestandteile einer Fermentationslösung bei der Aufreinigung von mikrobiell hergestellten Dihydroxyaceton, das in gelöster Form in der Fermentationslösung vorliegen.

Mikrobielle Fermentationsverfahren haben sich im technischen Maßstab schon seit vielen Jahren bewährt, da man hierdurch auf relativ einfache Art Zugang zu größeren Mengen an Substanzen hat, die mittels chemischer Synthese nicht, nur schwer oder nur in geringen Mengen zugänglich sind.

Das mittels mikrobieller Fermentation hergestellte Dihydroxyaceton liegt im Fermentationsmedium in der Regel in gelöster Form vor und muss aus diesem isoliert aund aufgereinigt werden. Im Labor- und Forschungsmaßstab stellt dies in der Regel kein größeres Problem dar, da genügend chromatographische Standardmethoden bekannt sind, Verunreinigungen abzutrennen. Im technischen Maßstab ist man jedoch auf einfache, schnelle und preiswerte Verfahren angewiesen, das gewünschte Produkt ohne weitere, größere Ausbeuteverluste in aufgereinigter Form zu isolieren, nach Möglichkeit auch ohne den allgemeinen kontinuierlichen Verfahrensprozeß wesentlich zu stören.

Ein besonderes Problem bei diesen Aufreinigungen ergibt sich aus der Tatsache, daß das Fermentationsmedium meist gefärbt ist. Die Färbung rührt einmal zum Teil von den fermentativen Bestandteilen des Kultivierungsmediums selbst her, andererseits läßt sie sich aber auch auf (unerwünscht) gebildete Zwischprodukte, Metaboliten oder Aggregate des Endproduktes zurückführen. In den aller meisten Fällen, kann die Färbung nicht zusammen durch das Abtrennen der gröberen Bestandteile inklusive der Zellen und Zelltrümmer des Mikroorganismus aus dem Medium nach abgeschlossener Fermentation beseitigt werden. Im technischen Maßstab wird heute zumeist Aktivkohle verwendet, um die unerwünschte Färbung zu entfernen. Die farbtragenden Stoffe werden dabei in der Regel an der Aktivkohle adsorbiert. Dieses Verfahren ist allerdings nur dann geeignet, wenn das gewünschte Produkt nicht ebenfalls adsorbiert wird. In vielen Fällen führt der Einsatz von Aktivkohle dennoch zu mehr oder weniger deutlichen Ausbeuteverlusten. Hinzukommt, daß die Aktivkohle meist in aufgeschlämmter Form zugeführt werden muß und in der Regel nach erfolgter Adsorption mehrfach ausgewaschen werden muß, um die Ausbeuteverluste zu minimieren. Dies hat einen in der Regel unerwünschten Verdünnungseffekt zur Folge.

Chemical Abstracts, vol. 112, no. 13,26.3.1990, Columbus, Ohio, US "Separation and recovery of fermented meso-erythritol with ultrafiltration membranes" beschreibt ein Verfahren zurTrennung und Isolierung von fermentiertem Mesoerythritol mittels Ultrafiltration mit Membranen, die eine Trenngrenze von 1000- 100.000 Molekulargewicht aufweisen, getrennt und isoliert. Das Filtrat wird anschließend mit Aktivkohle behandelt.

Die US 4,758,347 beschreibt ein Verfahren zur Reinigung/Entfärbung von farbstoffhaltigen Abwässern, die in einer Vorbehandlungsstufe durch Zugabe von Säure oder Alkali auf einen pH-Wert im Bereich von 4 bis 9 eingestellt sind, mit Hilfe eines zweistufigen Membrantrennverfahrens.

Die WO 91/04342 offenbart ein Verfahren zur Herstellung einer Mischung aus Fructose, Glucose und Verbindungen der allgemeinen Formel GFₙ, wobei G Glucose ist, F Fructose ist und n eine ganze Zahl ist, wobei die Mischung aus den Knollen des Topinambur oder aus Wurzeln der Zichorie durch ein Verfahren, das keinerlei chemische Modifizierung der Komponenten der Mischung beinhaltet, gewonnen wird.

Es bestand so die Aufgabe, die Nachteile, die bei der Entfärbung von Fermentationslösungen mit Aktivkohle im Zuge der Aufreinigung von mikrobiell hergestelltem Dihydroxyaceton auftreten, zu beseitigen.

Es wurde nun gefunden, daß die Verwendung von Aktivkohle zur Beseitigung von färbenden Begleitsubstanzen aus dem Medium vermieden werden kann, wenn sie durch eine Filtration durch geeignete Membranen ersetzt wird. Die Membranen müssen eine Trenngrenze von 150 bis maximal 500 u (150-500 Dalton) haben, daß heißt, Stoffe mit einem Molekulargewicht von deutlich unterhalb der Trenngrenze sind in der Lage, die Membranfläche zu permeieren, wohingegen Stoffe mit einem Molekulargewicht oberhalb der Trenngrenze zurückgehalten werden (Nanofiltration). Der Nanofiltration kann, wie bei der bekannten Verwendung von Aktivkohle auch, ein Filtrationsschritt vorausgehen, der bewirkt, daß die groben Bestandteile im Medium zuvor aus diesem entfernt werden (Mikrofiltration).

Gegenstand der vorliegenden Erfindung ist demnach ein Verfahren zur Aufreingung von mikrobiell hergestelltem Dihydroxyaceton in gefärbten Fermentationslösungen, das dadurch gekennzeichnet ist, daß man die von groben Bestandteilen befreite klare gefärbte Fermentationsbrühe, welche das mikrobiell her-gestellte Dihydroxyaceton in gelöster Form enthält, durch eine Membrane mit einer Trenngrenze von 200 bis 300 u (200-300 Dalton), filtriert und das Dihydroxyaceton aus dem ungefärbten Eluat isoliert.

Die für diese Zwecke erforderlichen Trennmembranen mit den genannten Eigenschaften sind Stand der Technik und kommerziell erwerbbar. Trennmembranen mit einer Trenngrenze von etwa 150 bis 500 u (150-500 Dalton) vermögen, die zum Teil unbekannten gelben und braunen gelösten Bestandteile einer ansonsten klaren Fermentationslösung nahezu quantitativ zurückzuhalten, während Syntheseprodukte mit einem Molekulargewicht von deutlich unterhalb der jeweiligen Trenngrenze in guten Ausbeuten durch die Membran permeieren. Insbesondere sind Trennmembranen geeignetmit einer Trenngrenze von 200 bis 300 u (200-300 Dalton). Je nach Größe des Molekulargewichts innerhalb des angegebenen Bereiches können 10 bis 30 % mehr Ausbeute (bezogen auf den Kristallisationsschritt im Anschluß an die Filtration bzw. die Kohleadsorption) mittels des erfindungsgemäßen Nanofiltrationsschrittes erreicht werden im Vergleich zur Aufreinigung mittels Aktivkohle unter ansonsten gleichen Vor- und Nachbehandlungen. Die entsprechenden Stoffe sollten vorzugsweise keine oder nur geringe Ladungen aufweisen, da Hydrathüllen zu einem größeren effektiven Molekulargewicht führen.

Auch wenn bei dem genannten Filtrationsschritt eine effektive Mindestmembranfläche vorhanden sein muß (bei z.B. für 5.000 I :100 - 140 qm) und Drucke (5 - 40 bar) erforderlich sind, die einen erhöhten apparativen und anfangs auch zeitlichen Aufwand zur Folge haben, ist ein Filtrationsschritt dem Einsatz von Aktivkohle nicht nur aus Gründen der genannten höheren Ausbeute vorzuziehen. So wird das Medium nicht mehr unnötig durch Wasch- und Aufschwämmlösung verdünnt, was sich störend auf die in der Regel sich noch anschließenden Chromatographieschritte, beispielsweise lonenaustauscherchromatographie, auswirkt. Auch die relativ langen Filtrierzeiten (5-10 h, je nachdem, welche Bedingungen vorliegen) sind letztlich kein eigentlicher Nachteil, da die Nanofiltration in ein kontinuierliches Verfahren zwischen Mikrofiltration (Entfernung von Zellen, groben Bestandteilen) und z.B lonenaustauscherchromatographie (zur weiteren Aufreinigung) eingebunden werden kann. Überdies werden Entsorgungsprobleme, die bei Verwendung von Aktivkohle massiv entstehen, umgangen.

Ein weiterer Vorteil des erfindungsgemäßen Vefahrens ist, daß neben der erhöhten Ausbeute das Produkt, welches sich aus der Fermentationslösung nach der Nanofiltration auskristallisieren läßt, eine bessere Qualität aufweist als bei der Behandlung mit Aktivkohle. So sind beispielsweise die sogenannten Gelbwerte, die sich von Lösungen auskristallisierten Produktes (2. Kristallisation) ermitteln lassen, deutlich niedriger als bei der Kohleadsorptionsmethode.

Weiterhin wurde festgestellt, daß entsprechende Lösungen (Filtermethode) einen wesentlich günstigeren pH-Wert (5.2 bis 6.0) aufweisen, als die nach der bekannten Methode (Kohleadsorption) aufgereinigten Produkte (<< 5.0). Dies wirkt sich überraschend und in vorteilhafter Weise auf eine verbesserte Lagerfähigkeit der endgültig aufgereinigten Produkte (zumeist ein Chromatographieschritt nach der Nanofiltration) aus.

Die Nanofiltration eignet sich bei allen gängigen technischen Fermentationsverfahren, unabhängig, welche Mikroorganismen und welche Medienzusammensetzungen vorliegen. Entscheidend ist letztlich die effektive Größe des gebildeten Produktes. Je weiter die Größe des Produktes unterhalb der Ausschlußgrenze liegt, mit um so höherern Ausbeuten eines besonders reiner Verbindung kann gerechnet werden. Die Nanofiltration kann auch ohne vorgeschaltete Mikrofiltration durchgeführt werden, so daß ein Aufreinigungsschritt eingespart werden kann.

Allerdings muß in diesem Fall eine größerer Membranfläche eingesetzt werden, und die Filtrationszeiten verängern sich entsprechend. Das Verfahren ist im besonderen Maße für die Aufreinigung von mikrobiell hergestelltem Dihydroxyaceton geeignet.

Die folgenden Beispiele erläutern die Erfindung.

### Beispiel 1:

Zur Umsetzung von Glycerin zu Dihydroxyaceton (DHA) mit Hilfe des Bakterienstammes *Gluconobacter oxydans* (ATCC 621) wurden 100 l eines Mediums mit folgender Zusammensetzung angesetzt:

| | |
|---|---|
| Glycerin | 150 g / l |
| Hefeextrakt | 5 g / l |
| Ammoniumsulfat | 2 g / l |

Die Lösung wurde sterilisiert (20 min, 120 °C) und nach dem Abkühlen mit 5 - 10 I einer dicht bewachsenen Vorkultur des Bakteriums beimpft.

Bei guter Belüftung (50 I/ min) und starker Durchmischung (500 Upm) im Fermenter ließ man die Bakterienkultur für ca. einen Tag wachsen und umsetzen. Die Dauer richtete sich dabei entweder nach der gemessenen Restglycerinmenge oder nach der Sauerstoffaufnahme, gemessen nach Standardmethoden. Die Umsetzung wurde abgebrochen, als kein Glycerin mehr im Ansatz vorhanden war.

### Beispiel 2:

Die nach Beispiel 1 erhaltene Fermentationsbrühe wird durch Mikrofiltration (Porengröße 0.2 µm) nach Standardmethoden von Feststoffen jeglicher Art befreit.

Das klare Filtrat wurde anschließend einer Nanofiltrationsanlage zugeführt:

| | |
|---|---|
| Trennmaterial | Dow NF 45, Trenngrenze 200 u (200 Da), |
| Membranfläche | 50 qm (Wickelmodule), |
| Druck | 20 bar, |
| Permeatfluß mit Filtrat von Beispiel 1 | 7 l / qm h. |

Bei 20 bar und turbulenter Querströmung (zur Vermeidung von Sedimentablagerungen) der Membranfläche wurde die Fermenterlösung filtriert. Dabei wurden die färbenden Bestandteile zurückgehalten, während Wasser und das Produkt die Membran permeierten. Die klare, ungefärbte DHA-Lösung wurde bis zur Zähflüssigkeit eingedampft. Aus diesem Konzentrat wurde das Produkt unter Kühlung aus Aceton auskristallisiert. Ausbeute 76 % weiße Kristalle. Einige Kristalle wurden in Wasser gelöst (25%ige Lösung) und die Gelbfärbung als Maß für noch vorhandene Verunreinigungen gemessen (nach Hazen A., 1892, Am. Chem. J. 14, 300). Es wurde ein relativer Gelbwert von 4.6 bestimmt. Der pH-Wert der Lösung betrug 5.6.

### Beispiel 3:

Die nach Beispiel 1 erhaltene Fermentationsbrühe wurde wie in Beispiel 2 beschrieben, zunächst der Mikrofiltration unterworfen.

Anschließend wurde das klare Filtrat mit ca. 2.5 kg Aktivkohle (aufgeschlämmt in 10 l Wasser) für 1 h in einem Behälter gerührt. Durch Filtration wurde die Kohle wieder entfernt. Die Kohle wurde 2mal mit wenig Wasser nachgewaschen. Aus der verbliebenen klaren ungefärbten DHA-Lösung wurde wie in Beispiel 2 beschrieben, DHA auskristallisiert. Ausbeute 61 %. Es wurde eine Gelbwertbestimmung der Lösung von Kristallen analog Beispiel 2 durchgeführt. Es wurde ein relativer Gelbwert von 15 ermittelt. Der pH-Wert der Lösung betrug 4.5.

## Patentansprüche

1. Verfahren zur Aufreinigung von mikrobiell hergestelltem Dihydroxyaceton in gefärbten Fermentationslösungen, **dadurch gekennzeichnet, dass** man die von groben Bestandteilen befreite klare gefärbte Fermentationsbrühe, welche das mikrobiell hergestellte Dihydroxyaceton in gelöster Form enthält, durch eine Membrane mit einer Trenngrenze von 200 bis 300 u (200-300 Dalton) filtriert und das Dihydroxyaceton aus dem ungefärbten Eluat isoliert.

## Claims

1. Process for purifying microbially produced dihydroxyacetone in coloured fermentation solutions, **characterized in that** the clear coloured fermentation broth which has been cleared of coarse components and which contains the microbially produced dihydroxyacetone in soluble form is filtered through a membrane having a cut-off of from 200 to 300 U (200-300 dalton) and the dihydroxyacetone is isolated from the uncoloured eluate.

## Revendications

1. Procédé pour la purification de dihydroxyacétone fabriquée par voie microbienne dans des solutions de fermentation colorées, **caractérisé en ce que** l'on filtre la bouillie de fermentation claire, colorée, débarrassée des éléments grossiers et contenant sous forme dissoute la dihydroxyacétone fabriquée par voie microbienne, à travers une membrane ayant un seuil de coupure compris entre 200 et 300 u (200 à 300 daltons) et **en ce que** l'on isole de l'éluat non coloré la dihydroxyacétone.
